# EUROPEAN PATENT SPECIFICATION

(11) **EP 3 025 684 B1**
(45) Date of publication and mention of the grant of the patent: **04.10.2017**
(21) Application number: 15192537.7
(22) Date of filing: 02.11.2015
(51) Int. Cl.: A61F 2/848, A61F 2/86, A61F 2/90

(54) **SELF-EXPANDABLE STENT**
SELBSTEXPANDIERBARER STENT
STENT AUTO-EXTENSIBLE

(30) Priority: 26.11.2014 JP 2014238858
(43) Date of publication of application: 01.06.2016
(73) Proprietor: PENTAS Inc., Tokyo, 150-0002 (JP)
(72) Inventor: NISHIGISHI, Makoto, Nagoya-shi, Aichi 463-0024 (JP)
(74) Representative: TBK

(56) References cited:
- WO-A1-97/16133
- WO-A1-2012/082440
- US-A1- 2010 217 381

## Description

### TECHNICAL FIELD

The present invention relates to a self-expandable stent for supporting the lumen of a blood vessel or digestive organ in order to prevent restenosis of a narrowed or obstructed segment formed in the blood vessel or digestive organ, or for confining an embolization coil in an aneurysm formed in an arterial vessel of the abdomen or the brain to prevent the aneurysm from rupturing.

### BACKGROUND ART

Stents are medical devices used for treating a stenosis or occlusion and an aneurysm formed in a blood vessel or digestive organ (hereinafter referred to as a "target site"). Stents are broadly classified into two categories: a balloon-expandable stent which is extendable with a balloon catheter and a self-expandable stent in which the stent itself expands spontaneously. Recently, self-expandable stents have been often used which are resistant to deformation even when external force is applied.

In the case of a self-expandable stent, the stent is usually released at the target site from the distal end of a catheter by pushing a pusher guide wire in the direction of the distal end after delivered to the target site. In order to prevent dislocation from the target site at this time, an anchor member is attached to an end of a self-expandable stent (for example, see Patent Literatures 1 and 2 below). The anchoring effect of an anchor member on the wall of a blood vessel wall or digestive organ can prevent a self-expandable stent from moving axially or rotating radially when the self-expandable stent is expanded at the target site.

However, for the self-expandable stents according to Patent Literatures 1 and 2, an anchor member does not have a degree of freedom against an wire since both the distal end and the proximal end of the anchor member are fixed to the wire of the self-expandable stent. Therefore, the anchor member can not easily make contact with a blood vessel wall or digestive organ wall at the optimal position when the self-expandable stent is expanded at the target site. As a result, the anchor effect of the anchor member on the blood vessel wall or digestive organ wall is insufficient, and thus the self-expandable stent may be disadvantageously dislocated from the target site. In particular, a gap between the anchor member and the blood vessel wall or digestive organ wall may be formed when the target site is located at a curved portion, resulting in a significant problem in which the anchor effect of the anchor member on the blood vessel wall or digestive organ wall is insufficient. A self-expanding stent with anchor members is shown in US 2010/0217381

### CITATION LIST

### Patent Literature

Patent Literature 1: JP 2014-513987
Patent Literature 2: JP 4672241

### SUMMARY OF THE INVENTION

Insofar as the terms "invention" and/or "embodiment" are used in the following, and/or features are presented as being optional, this should be interpreted in such a way that the only protection sought is that of the invention as claimed.

### TECHNICAL PROBLEM

The present invention is made in view of these circumstances. An object of the present invention is to provide a self-expandable stent in which an anchor member makes contact with a blood vessel wall or digestive organ wall at the optimal position, and a gap is not easily formed between the anchor member and the blood vessel wall or digestive organ wall, and as a result, the self-expandable stent is not easily dislocated from the target site when the self-expandable stent is expanded at the target site.

### SOLUTION TO PROBLEM

The above object can be achieved by the means listed below,

An aspect 1 of the present invention is an self-expandable stent comprising a plurality of first wires; a plurality of second wires interwoven with the plurality of first wires; and a first anchor member attached to a distal end of one wire of the plurality of first wires and the plurality of second wires, wherein a distal end of the first anchor member is fixed to the one wire, while a proximal end of the first anchor member is a free end and not fixed to the one wire.

The term "a first anchor member" may refer to a single first anchor member or one of a plurality of first anchor members. In the case of the plurality of first anchor members, the first anchor members are disposed at a same position in an axial direction, and each of the first anchor members is attached to a distal end of respective one of the plurality of first wires and the plurality of second wires.

An aspect 2 of the present invention is the self-expandable stent according to the aspect 1, further comprising a second anchor member attached to a distal end of a different wire of the plurality of first wires and the plurality of second wires, that is different from the one wire to which the first anchor member is attached, wherein a distal end of the second anchor member is fixed to the different wire, while a proximal end of the second anchor member is a free end and not fixed to the different wire, and the second anchor member is disposed at a position different from the first anchor member in an axial direction.

The term "a second anchor member" may refer to a single second anchor member or one of a plurality of second anchor members. In the case of the plurality of second anchor members, the second anchor members are disposed at a same position in an axial direction, and each of the second anchor members is attached to a distal end of respective one of the plurality of first wires and the plurality of second wires, that is different from the one wire to which the first anchor member is attached.

An aspect 3 of the present invention is the self-expandable stent according to the aspect 1 or 2, further comprising a third anchor member attached to a proximal end of one wire of the plurality of first wires and the plurality of the second wires, wherein a distal end of the third anchor member is fixed to the one wire, while a proximal end of the third anchor member is a free end and not fixed to the one wire.

The term "a third anchor member" may refer to a single third anchor member or one of a plurality of third anchor members. In the case of the plurality of third anchor members, the third anchor members are disposed at a same position in an axial direction, and each of the third anchor members is attached to a proximal end of respective one of the plurality of first wires and the plurality of the second wires.

Another aspect of the present invention is the self-expandable stent according to any one of the above aspects, wherein a tensile strength of each of the plurality of first wires is higher than a tensile strength of each of the plurality of second wires. In this aspect of the present invention, the first anchor member, the second anchor member, and the third anchor member are preferably attached to the first wires.

Another aspect of the present invention is the self-expandable stent according to any one of the above aspects, wherein a center of the one wire is displaced from a center of the first anchor member, as viewed at a horizontal cross section orthogonal to the axial direction of the one wire.

In the present invention, each of the first anchor member, the second anchor member and the third anchor member may be a tube body, a hollow coil body, or, in more general terms, a hollow cylinder body a distal end of which is fixed to a respective one wire of the plurality of first wires and the plurality of second wires. The distal end of the hollow cylinder body may be a closed distal end, i.e., the hollow cylinder body may be a cap-shaped hollow cylinder body. In any case the hollow cylinder body may be fixed to the respective one wire of the plurality of first wire and the plurality of second wires by inserting a distal end of the respective one wire into the hollow cylinder body and then fixing the distal end of the respective one wire and the distal end of the hollow cylinder body to each other, e.g. by laser irradiation or using an adhesive.

### ADVANTAGEOUS EFFECT OF THE INVENTION

In the self-expandable stent according to the aspect 1 of the present invention, a distal end of the first anchor member is fixed to the one wire while a proximal end of the first anchor member is a free end and not fixed to the one wire. The first anchor member has a degree of freedom against the one wire, and the proximal end of the first anchor member can pivot on the distal end. Therefore, when the self-expandable stent is expanded at the target site, the first anchor member can easily make contact with a blood vessel wall or digestive organ wall at the optimal position. In particular, in a case where the target site is located at a curved portion, the first anchor member can make contact at the optimal position depending on the curvature of the blood vessel wall or digestive organ wall. Therefore, a gap is not easily formed between the anchor member and the blood vessel wall or digestive organ wall. As a result, the anchor effect of the first anchor member on the blood vessel wall or digestive organ wall can be improved, reducing a risk that the self-expandable stent is axially or radially dislocated from the target site.

In the self-expandable stent according to the aspect 2 of the present invention, a second anchor member is attached to the distal end of a different wire that is different from the one wire to which the first anchor member is attached. A distal end of the second anchor member is fixed to the different wire while a proximal end of the second anchor member is a free end and not fixed to the different wire. The second anchor member is disposed at a position different from the first anchor member in the axial direction. The anchor effect on a blood vessel wall or digestive organ wall can be improved in the axial direction by disposing the second anchor member having a degree of freedom at an axially different position in addition to the first anchor member, reducing a risk that the self-expandable stent is axially or radially dislocated from the target site.

In the self-expandable stent according to another aspect of the present invention, a tensile strength of each of the plurality of first wires is higher than a tensile strength of each of the plurality of second wires. Therefore, the first anchor member attached to the one wire of the plurality of first wires with high tensile strength can make contact with a blood vessel wall or digestive organ wall preferentially to the plurality of second wires with low tensile strength when the self-expandable stent is expanded at the target site. Thereby, damages to the blood vessel wall or digestive organ wall by the distal end of each of the plurality of second wires to which the anchor member is not attached can be reduced while maintaining the anchor effect of the first anchor member on the blood vessel wall or digestive organ wall.

In the self-expandable stent according to another aspect of the present invention, a center of the one wire is displaced from a center of the first anchor member, as viewed at a horizontal cross section orthogonal to the axial direction of the one wire. The degree of freedom of the first anchor member becomes further larger, and the range of motion of the proximal end of the first anchor pivoting on the distal end can be increased. Therefore, even in a case where a blood vessel wall or digestive organ wall has an uneven surface due to a plaque and the like, contact can be made at the optimal position depending on the uneven surface. As a result, a risk can be further reduced that a self-expandable stent is axially or radially dislocated from the target site regardless of the conditions of a blood vessel wall or digestive organ wall at the target site.

### BRIEF DESCRIPTION OF THE DRAWINGS

Fig. 1 shows an overall view of a self-expandable stent according to an embodiment of the present invention.
Fig. 2 shows an enlarged cross sectional view of Portion A in Fig. 1.
Fig. 3 shows a self-expandable stent stored inside a catheter which is delivered near the target site.
Fig. 4 shows a self-expandable stent released at the target site to make contact with a blood vessel wall or digestive organ wall.
Fig. 5 shows an overall view of a self-expandable stent according to the second embodiment.
Fig. 6 shows a part of a self-expandable stent according to the third embodiment which is a first variation of Fig. 2.
Fig. 7A shows a part of a self-expandable stent according to the fourth embodiment which is a second variation of Fig. 2. Fig. 7B shows the B-B cross section of Fig. 7A.
Fig. 8 shows an overall view of a self-expandable stent according to the fifth embodiment.

### DESCRIPTION OF EMBODIMENTS

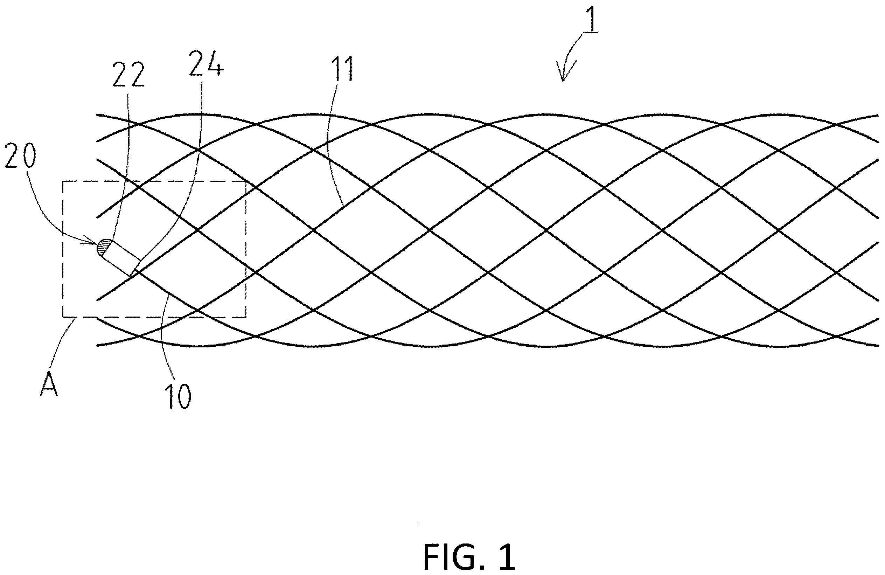

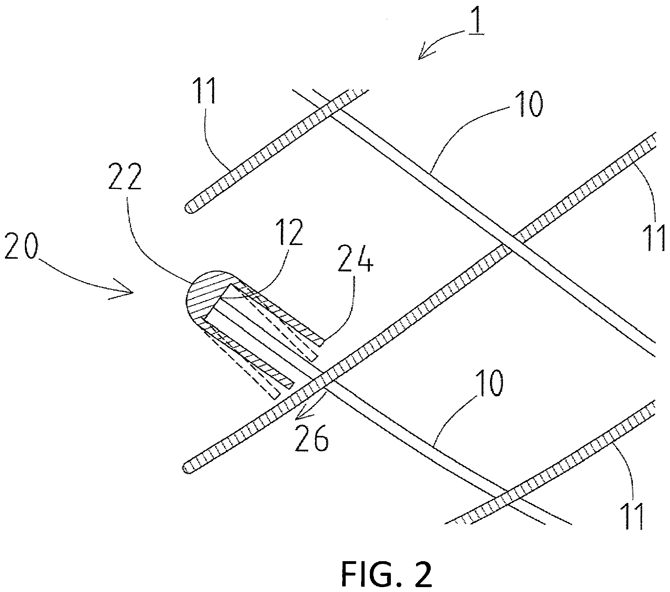

A use of a self-expandable stent 1 according to an embodiment of the present invention is described with reference to Figs. 1 to 4. The left side in Figs. 1 to 4 corresponds to the distal side (the front end side) which is to be inserted into the body, and the right side corresponds to the proximal side (the base end side) which is to be operated by an operator such as a physician. Note that Fig. 2 is an enlarged cross sectional view of Section A in Fig. 1.

The self-expandable stent 1 is used, for example, to treat a target site formed at a blood vessel wall or digestive organ wall. As shown in Figs. 1 and 2, the self-expandable stent 1 comprises a plurality of first wires 10 and a plurality of second wires 11 interwoven with the plurality of first wires 10 to form a mesh-like (a net-like) structure.

In this embodiment of the present invention, the plurality of first wires 10 and the plurality of second wires 11 are made of, but not limited to, a Co-Cr alloy with high tensile strength. For example, the plurality of first wires 10 and the plurality of second wires 11 may be made of a metal material such as stainless steel, W, Pt, a Pt-Ni alloy, a Ni-Ti alloy and a Cu-Al-Ni alloy; and a resin material such as polyester, polyurethane, polyolefine, polytetrafluoroethylene, a silicon resin.

Further, in this embodiment of the present invention, eight first wires 10 wind in the clockwise direction, and eight second wires 11 wind in the anticlockwise direction, and thus a total of 16 (8 x 8) wires 10, 11 are interwoven each other. However, the number of the wires 10, 11 in the self-expandable stent 1 is not be limited to 16 (8 x 8) in total, but may be 24 (12 x 12) in total or 32 (16 x 16) in total.

As shown in Fig. 2, a single first anchor member 20 is attached to a distal end 12 of one of the plurality of first wires 10. A distal end 22 of the first anchor member 20 is fixed to the distal end 12 of one of the plurality of first wires 10, but a proximal end 24 is a free end and not fixed to the one of the plurality of first wires 10. Therefore, the first anchor member 20 has a degree of freedom against the one of the plurality of first wires 10, and for example, the proximal end 24 can pivot on the distal end 22 like a pendulum in the direction of an arrow 26.

In this embodiment of the present invention, the first anchor member 20 is a tube body made of the same material as the first wires 10. However, there is no particular limitation for the material of the first anchor member 20, and, for example, a material different from that of the first wires 10 may be used. Further, the first anchor member 20 may be formed into a hollow coil body or, in more general terms, a hollow cylinder body.

In the self-expandable stent 1, the first anchor member 20 is attached by inserting the distal end 12 of the one of the plurality of first wires 10 into the hollow cylinder body, and then irradiating the distal end 12 of the one of the plurality of first wires 10 and the distal end of the hollow cylinder body with laser. However, it is not limited to this, and the first anchor member 20 may be attached to the distal end 12 of the one of the plurality of first wires 10 with an adhesive.

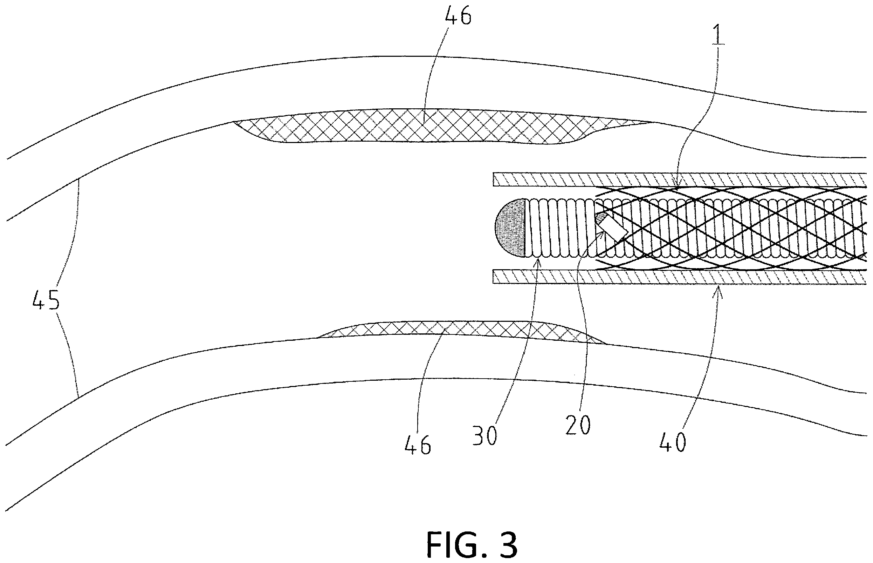

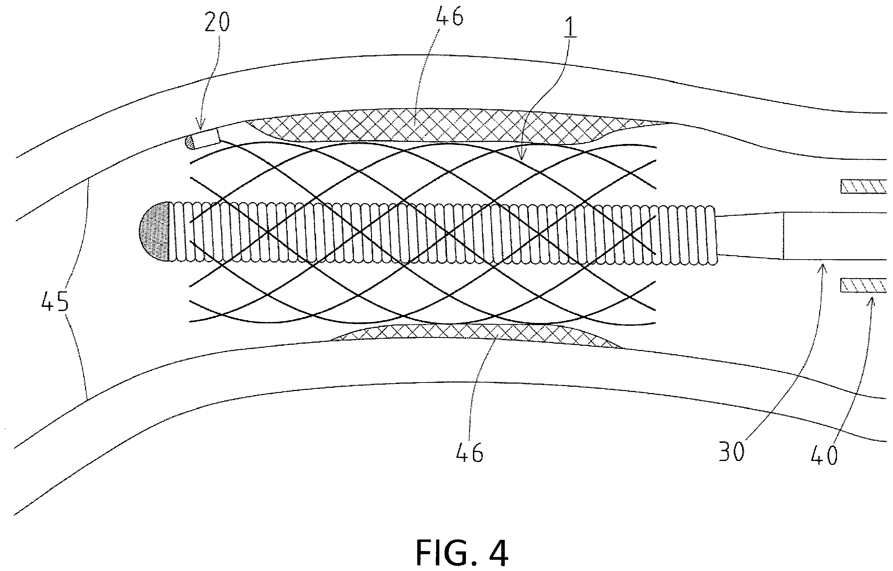

As shown in Fig. 3, the self-expandable stent 1 stored inside a catheter 40 is delivered to the near side of the target site 46 formed on a blood vessel wall or digestive organ wall 45. Then, as shown in Fig. 4, the self-expandable stent 1 is released from the distal end of the catheter 40 to the target site 46 by pushing a pusher guide wire 30 in the direction of the distal end while pulling the catheter 40 in the direction of the proximal end. Thereby, the self-expandable stent 1 can support the lumen of the blood vessel or digestive organ to prevent restenosis of the target site 46 formed on the blood vessel wall or digestive organ wall 45.

At this time, the first anchor member 20 has a degree of freedom against the one of the plurality of first wires 10. When the self-expandable stent 1 is expanded at the target site 46, the proximal end 24 of the first anchor member 20 can pivot on the distal end 22, and the first anchor member 20 can make contact with the blood vessel wall or digestive organ wall 45 at the optimal position. Even in a case where the target site 46 is located at a curved portion, the first anchor member 20 can make contact at the optimal position depending on the curvature of the blood vessel wall or digestive organ wall 45. Therefore, a gap is not easily formed between the first anchor member 20 and the blood vessel wall or digestive organ wall 45. As a result, the anchor effect of the first anchor member 20 on the blood vessel wall or digestive organ wall 45 can be improved, reducing a risk that the self-expandable stent 1 is axially or radially dislocated from the target site 46.

In this embodiment of the present invention, a single first anchor member 20 is attached to the distal end 12 of one of the plurality of first wires 10, but the configuration is not limited to this. For example, a first anchor member 20 may be attached to a distal end of one of the plurality of second wires 11, or first anchor members 20 may be attached to each of the distal end of one of the plurality of first wires 10 and one of the plurality of second wires 11.

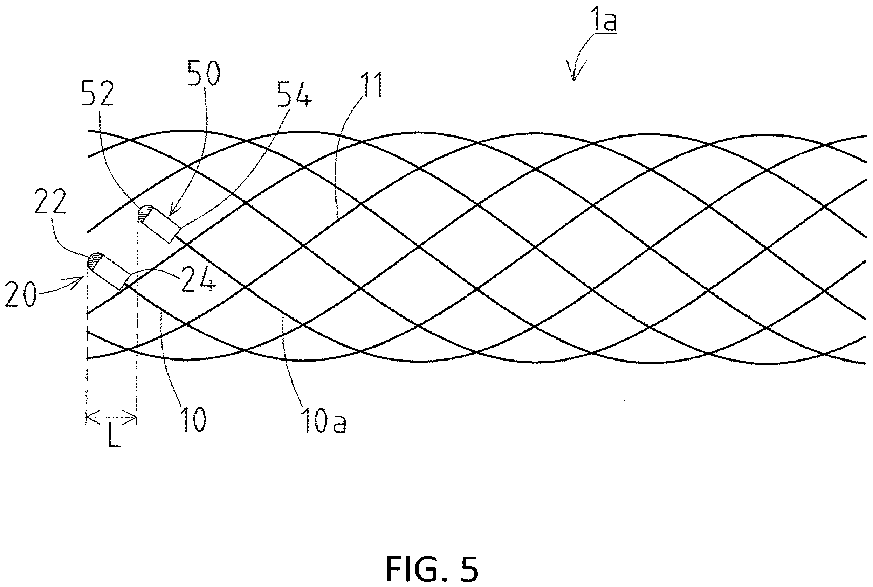

Next, a self-expandable stent 1a according to the second embodiment is described with reference to Fig. 5. Note that as in Figs. 1 to 4, the left side in Figs. 5 corresponds to the distal side (the front end side) which is to be inserted into the body, and the right side corresponds to the proximal side (the base end side) which is to be operated by an operator such as a physician.

Only differences from the self-expandable stent 1 shown in Fig. 1 will be described. As shown in Fig. 5, in the self-expandable stent 1a, a second anchor member 50 is attached to a distal end 12a of a different wire 10a that is different from the one of the plurality of first wires 10 to which the first anchor member 20 is attached. A distal end 52 of the second anchor member 50 is fixed to the different wire 10a while a proximal end 54 is a free end and not fixed to the different wire 10a. Therefore, the second anchor member 50 has a degree of freedom against the different wire 10a as in the first anchor member 20. For example, the proximal end 54 can pivot on the distal end 52 like a pendulum.

The second anchor member 50 is disposed at a position different from the first anchor member 20 in the axial direction. As shown in Fig. 5, the second anchor member 50 is separated by a distance L toward the proximal side relative to the first anchor member 20. As described above, in the self-expandable stent 1a, the second anchor member 50 having a degree of freedom is disposed at an axially different position in addition to the first anchor member 20. Therefore, the anchor effect on a blood vessel wall or digestive organ wall can be improved in the axial direction, further reducing a risk that the self-expandable stent 1a is axially dislocated from the target site.

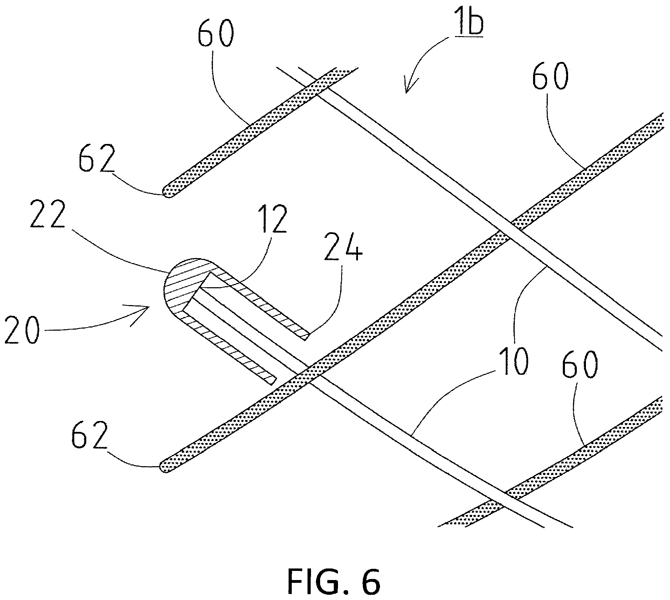

Next, a self-expandable stent 1b according to the third embodiment is described with reference to Fig. 6. Note that as in Figs. 1 to 4, the left side in Figs. 6 corresponds to the distal side (the front end side) which is to be inserted into the body, and the right side corresponds to the proximal side (the base end side) which is to be operated by an operator such as a physician. Note that Fig. 6 corresponds to a first variation of Fig. 2.

Only differences from the self-expandable stent 1 shown in Fig. 2 will be described. As shown in Fig. 6, in the self-expandable stent 1b, the plurality of first wires 10 with high tensile strength and a plurality of second wires 60 with low tensile strength are interwoven each other to form a mesh-like (net-like) structure. The first anchor member 20 is attached to the distal end 12 of one of the plurality of first wires 10 with high tensile strength. In construct, the first anchor member 20 is not attached to a distal end 62 of one of the plurality of second wires 60 with low tensile strength.

When the self-expandable stent 1b is expanded at the target site, the first anchor member 20 attached to the one of the plurality of first wires 10 with high tensile strength can make contact with a blood vessel wall or digestive organ wall preferentially to the plurality of second wires 60 with low tensile strength. This can reduce damages on the blood vessel wall or digestive organ wall due to the distal end 62 of each of the plurality of second wires 60 to which the anchor member 20 is not attached while maintaining the anchor effect of the first anchor member 20 on the blood vessel wall or digestive organ wall.

In the self-expandable stent 1b, the plurality of first wires 10 are made of a Co-Cr alloy with high tensile strength, and the plurality of second wires 60 are made of a Pt-Ni alloy with low tensile strength. Since the Pt-Ni alloy has radiopacity, an operator such as a physician can precisely detect the position of the self-expandable stent 1b when the X-ray imaging.

Note that in the self-expandable stent 1b, the tensile strength of the plurality of first wires 10 is higher than that of the plurality of second wires 60 because a metal material of the plurality of first wires 10 has higher tensile strength than that of the plurality of second wires 60. However, the materials used for the plurality of first wires 10 and the plurality of second wires 60 are not limited to metal materials, and resin materials may be used as long as the tensile strength of the plurality of first wires 10 will be higher than that of the plurality of second wires 60.

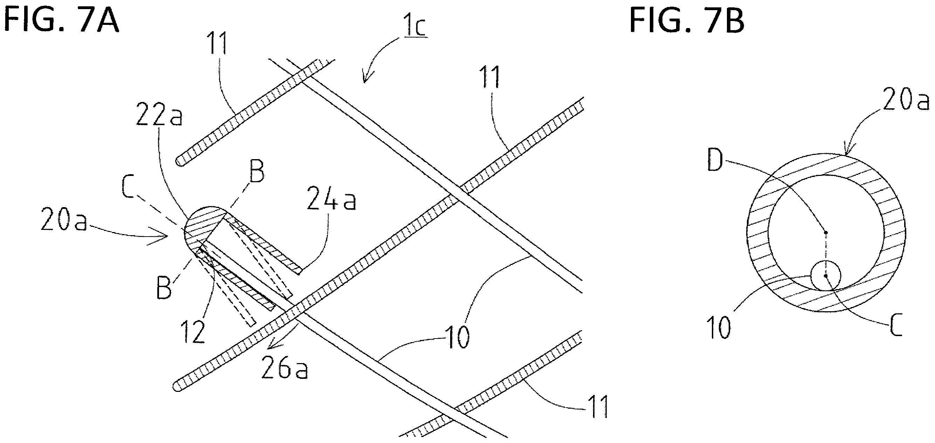

Next, a self-expandable stent 1c according to the fourth embodiment is described with reference to Figs. 7A and 7B. Note that as in Figs. 1 to 4, the left side in Figs. 7A corresponds to the distal side (the front end side) which is to be inserted into the body, and the right side corresponds to the proximal side (the base end side) which is to be operated by an operator such as a physician. Note that Fig. 7A corresponds to a second variation of Fig. 2.

As shown in Fig. 7A, in the self-expandable stent 1c, a first anchor member 20a is attached to the distal end 12 of the one of the plurality of first wires 10 as in the self-expandable stent 1, and a distal end 22a of the first anchor member 20a is fixed to the distal end 12 of the one of the plurality of first wires 10, and a proximal end 24a is a free end and not fixed to the one of the plurality of first wires 10.

Fig. 7B shows the B-B cross section in Fig. 7A (in other words, as viewed at a horizontal cross section orthogonal to an axis direction C of the plurality of first wires 10.) As shown in Fig. 7B, the center C of the one of the plurality of first wires 10 is displaced from the center D of the first anchor member 20a. Therefore, the degree of freedom of the first anchor member 20a can be further increased. For example, the proximal end 24a can widely pivot on the distal end 22a in the direction of an arrow 26a like a pendulum. Therefore, even in a case where a blood vessel wall or digestive organ wall has an uneven surface due to a plaque and the like, a contact can be made at the optimal position depending on the uneven surface. As a result, a risk can be further reduced that the self-expandable stent 1c is axially or radially dislocated from the target site regardless of the conditions of a blood vessel wall or digestive organ wall at the target site.

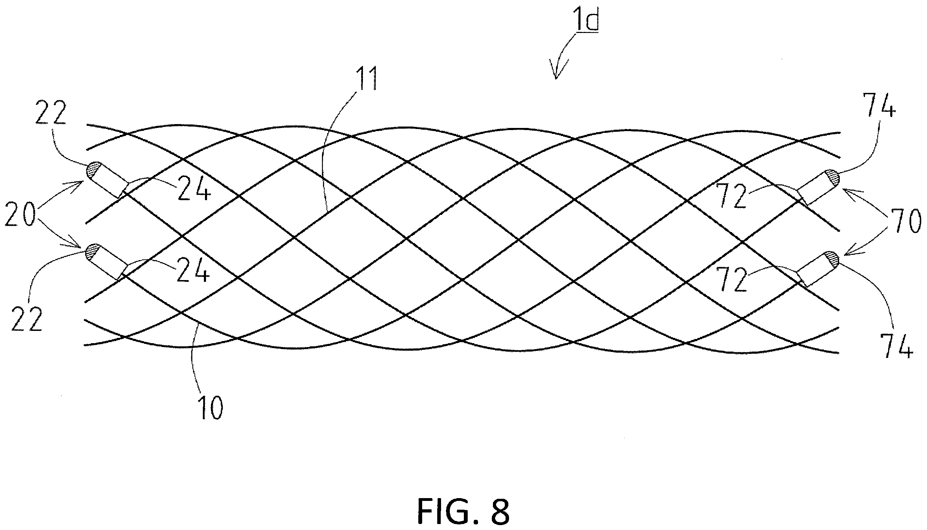

Finally, a self-expandable stent 1d according to the fifth embodiment is described with reference to Fig. 8. Note that as in Figs. 1 to 4, the left side in Figs. 8 corresponds to the distal side (the front end side) which is to be inserted into the body, and the right side corresponds to the proximal side (the base end side) which is to be operated by an operator such as a physician.

Only differences from the self-expandable stent 1 shown in Fig. 1 will be described. As shown in Fig. 8, in the self-expandable stent 1d, a plurality of first anchor members 20 are attached to the distal ends 12 of the plurality of first wires 10, and a plurality of third anchor members 70 are attached to proximal ends 14 of the plurality of first wires 10.

Distal ends 74 of the third anchor members 70 are fixed to the proximal ends of the plurality of first wires 10, and proximal ends 72 are free ends and not fixed to the plurality of first wires 10. Therefore, the third anchor members 70 have a degree of freedom against the plurality of first wires 10 as in the first anchor member 20. For example, the proximal ends 72 can pivot on the distal ends 74 like a pendulum.

In the self-expandable stent 1d, the plurality of first anchor members 20 and the plurality of third anchor members are provided at the distal end side and the proximal end side of the plurality of first wires 10, respectively. Therefore, the anchor effect on a blood vessel wall or digestive organ wall can be improved, further reducing a risk that the self-expandable stent 1d is axially or radially dislocated from the target site.

Note that the aforementioned self-expandable stents 1, 1a, 1b, 1c and 1d each have a cylindrical shape with a constant outer diameter in the axial direction, but the configuration is not limited to this. For example, the self-expandable stents 1, 1a, 1b, 1c and 1d each may have a small outer diameter at the central part while the self-expandable stents 1, 1a, 1b, 1c and 1d may each have a large outer diameter at the both ends (in other words, the self-expandable stents 1, 1a, 1b, 1c and 1d each may have a flared distal end side and flared proximal end side).

As described above, in the self-expandable stents 1, 1a, 1b, 1c and 1d, the first anchor member 20, 20a is attached to the distal end 12 of the one of the plurality of first wires 10. The distal end 22 or 22a of the first anchor member 20 or 20a is fixed to the one of the plurality of first wires 10, and the proximal ends 24 and 24a are free ends and not fixed to the one of the plurality of first wires 10. Therefore, when the self-expandable stents 1, 1a, 1b, 1c and 1d are expanded at the target site, a gap is not easily formed between the first anchor member 20, 20a and a blood vessel wall or digestive organ wall. As a result, the anchor effect of the first anchor member 20, 20a on the blood vessel wall and digestive organ wall can be improved, reducing a risk that the self-expandable stents 1, 1a, 1b, 1c and 1d are axially or radially dislocated from the target site.

### Description of Symbols

- 1, 1a, 1b, 1c, 1d: Self-expandable stent
- 10, 10a: First wire
- 11, 60: Second wire
- 12: Distal end of the first wire
- 14: Proximal end of the first wire
- 20, 20a: First anchor member
- 22, 22a: Distal end of the first anchor member
- 24, 24a: Proximal end of the first anchor member
- 26, 26a: Movable direction
- 30: Pusher guide wire
- 40: Catheter
- 45: Blood vessel wall or digestive organ wall
- 46: Target site
- 50: Second anchor member
- 52: Distal end of the second anchor member
- 54: Proximal end of the second anchor member
- 62: Distal end of the second wire
- 70: Third anchor member
- 72: Distal end of the third anchor member
- 74: Proximal end of the third anchor member

## Claims

1. A self-expandable stent (1, 1a, 1b, 1c, 1d) comprising:
a plurality of first wires (10, 10a);
a plurality of second wires (11, 60) interwoven with the plurality of first wires (10, 10a); and
a first anchor member (20, 20a) attached to a distal end (22, 22a) of one wire of the plurality of first wires (10) and the plurality of second wires (11, 60),
**characterized in that**:
a distal end (22, 22a) of the first anchor member (20, 20a) is fixed to the one wire, while a proximal end (24, 24a) of the first anchor member (20, 20a) is a free end and not fixed to the one wire.

2. The self-expandable stent (1a) according to claim 1, further comprising:
a second anchor member (50) attached to a distal end (12) of a different wire (10a) of the plurality of first wires (10) and the plurality of second wires (11, 60), that is different from the one wire to which the first anchor member (20) is attached, wherein
a distal end (52) of the second anchor member (50) is fixed to the different wire (10a), while a proximal end (54) of the second anchor member (50) is a free end and not fixed to the different wire (10a), and
the second anchor member (50) is disposed at a position different from the first anchor member (20) in an axial direction.

3. The self-expandable stent (1d) according to claim 1 or 2, further comprising:
a third anchor member (70) attached to a proximal end of one wire of the plurality of first wires (10) and the plurality of second wires (11, 60), wherein
a distal end (72) of the third anchor member (70) is fixed to the one wire, while a proximal end (74) of the third anchor member (70) is a free end and not fixed to the one wire.

4. The self-expandable stent (1, 1a, 1b, 1c, 1d) according to any one of claims 1 to 3, wherein the one wire, to which the first anchor member (20) is attached, is one of the plurality of first wires (10).

5. The self-expandable stent (1a) according to claim 4, wherein the different wire, to which the second anchor member (50) is attached, is one of the plurality of first wires (10).

6. The self-expandable stent (1d) according to any one of claims 4 or 5, wherein the one wire, to which the third anchor member (70) is attached, is one of the plurality of first wires (10).

7. The self-expandable stent (1b) according to any one of claims 4 to 6, wherein
a tensile strength of each of the plurality of first wires (10) is higher than a tensile strength of each of the plurality of second wires (60).

8. The self-expandable stent (1c) according to any one of claims 1 to 7, wherein
a center (C) of the one wire is displaced from a center (D) of the first anchor member (20a), as viewed at a horizontal cross section orthogonal to the axial direction of the one wire.

9. The self-expandable stent (1b) according to claim 7, wherein
the plurality of first wires are made of a Co-Cr alloy, and
the plurality of second wires are made of a Pt-Ni alloy.

## Patentansprüche

1. Selbstexpandierbarer Stent (1, 1a, 1b, 1c, 1d), aufweisend:
eine Vielzahl erster Drähte (10, 10a);
eine Vielzahl zweiter Drähte (11, 60), die mit der Vielzahl erster Drähte (10, 10a) verflochten ist; und
ein erstes Ankerelement (20, 20a), das an einem Distalende (22, 22a) eines Drahts der Vielzahl erster Drähte (10) und der Vielzahl zweiter Drähte (11, 60) angebracht ist,
**dadurch gekennzeichnet, dass**:
ein Distalende (22, 22a) des ersten Ankerelements (20, 20a) an dem einen Draht befestigt ist, während ein Proximalende (24, 24a) des ersten Ankerelements (20, 20a) ein freies Ende ist und nicht an dem einen Draht befestigt ist.

2. Selbstexpandierbarer Stent (1a) nach Anspruch 1, ferner aufweisend:
ein zweites Ankerelement (50), das an einem Distalende (12) eines anderen Drahts (10a) der Vielzahl erster Drähte (10) und der Vielzahl zweiter Drähte (11, 60) angebracht ist, der anders ist als der eine Draht, an dem das erste Ankerelement (20) angebracht ist, wobei
ein Distalende (52) des zweiten Ankerelements (50) an dem anderen Draht (10a) befestigt ist, während ein Proximalende (54) des zweiten Ankerelements (50) ein freies Ende ist und nicht an dem anderen Draht (10a) befestigt ist, und
das zweite Ankerelement (50) an einer Position in einer Axialrichtung angeordnet ist, die anders ist als die des ersten Ankerelements (20).

3. Selbstexpandierbarer Stent (1d) nach Anspruch 1 oder 2, ferner aufweisend:
ein drittes Ankerelement (70), das an einem Proximalende eines Drahts der Vielzahl erster Drähte (10) und der Vielzahl zweiter Drähte (11, 60) angebracht ist, wobei
ein Distalende (72) des dritten Ankerelements (70) an dem einen Draht befestigt ist, während ein Proximalende (74) des dritten Ankerelements (70) ein freies Ende ist und nicht an dem einen Draht befestigt ist.

4. Selbstexpandierbarer Stent (1, 1a, 1b, 1c, 1d) nach einem der Ansprüche 1 bis 3, wobei der eine Draht, an dem das erste Ankerelement (20) angebracht ist, einer der Vielzahl erster Drähte (10) ist.

5. Selbstexpandierbarer Stent (1a) nach Anspruch 4, wobei der andere Draht, an dem das zweite Ankerelement (50) angebracht ist, einer der Vielzahl erster Drähte (10) ist.

6. Selbstexpandierbarer Stent (1d) nach einem der Ansprüche 4 oder 5, wobei der eine Draht, an dem das dritte Ankerelement (70) angebracht ist, einer der Vielzahl erster Drähte (10) ist.

7. Selbstexpandierbarer Stent (1d) nach einem der Ansprüche 4 bis 6, wobei
eine Zugfestigkeit jedes der Vielzahl erster Drähte (10) höher ist als eine Zugfestigkeit jedes der Vielzahl zweiter Drähte (60).

8. Selbstexpandierbarer Stent (1c) nach einem der Ansprüche 1 bis 7, wobei
eine Mitte (C) des einen Drahts von einer Mitte (D) des ersten Ankerelements (20a), in einem horizontalen Querschnitt betrachtet, der senkrecht zu der Axialrichtung des einen Drahts ist, versetzt ist.

9. Selbstexpandierbarer Stent (1b) nach Anspruch 7, wobei die Vielzahl erster Drähte aus einer Co-Cr-Legierung gemacht ist, und die Vielzahl zweiter Drähte aus einer Pt-Ni-Legierung gemacht ist.

## Revendications

1. Stent auto-expansible (1, 1a, 1b, 1c, 1d) comprenant :
une pluralité de premiers fils (10, 10a) ;
une pluralité de seconds fils (11, 60) entrelacée avec la pluralité de premiers fils (10, 10a) ; et
un premier élément d'ancrage (20, 20a) fixé à une extrémité distale (22, 22a) d'un fil de la pluralité de premiers fils (10) et de la pluralité de seconds fils (11, 60),
**caractérisé en ce que** :
une extrémité distale (22, 22a) du premier élément d'ancrage (20, 20a) est fixée au un fil, alors qu'une extrémité proximale (24, 24a) du premier élément d'ancrage (20, 20a) est une extrémité libre et non fixée au un fil.

2. Stent auto-expansible (1a) selon la revendication 1, comprenant en outre :
un deuxième élément d'ancrage (50) fixé à une extrémité distale (12) d'un fil différent (10a) de la pluralité de premiers fils (10) et de la pluralité de seconds fils (11, 60) qui est différent du un fil auquel le premier élément d'ancrage (20) est fixé, dans lequel :
une extrémité distale (52) du deuxième élément d'ancrage (50) est fixée au fil différent (10a), alors qu'une extrémité proximale (54) du deuxième élément d'ancrage (50) est une extrémité libre et non fixée au fil différent (10a), et
le deuxième élément d'ancrage (50) est disposé dans une position différente du premier élément d'ancrage (20) dans une direction axiale.

3. Stent auto-expansible (1d) selon la revendication 1 ou 2, comprenant en outre :
un troisième élément d'ancrage (70) fixé à une extrémité proximale du un fil de la pluralité de premiers fils (10) et de la pluralité de seconds fils (11, 60), dans lequel :
une extrémité distale (72) du troisième élément d'ancrage (70) est fixée sur le un fil, alors qu'une extrémité proximale (74) du troisième élément d'ancrage (70) est une extrémité libre et non fixée au un fil.

4. Stent auto-expansible (1, 1a, 1b, 1c, 1d) selon l'une quelconque des revendications 1 à 3, dans lequel le un fil, auquel le premier élément d'ancrage (20) est fixé, est l'un de la pluralité de premiers fils (10).

5. Stent auto-expansible (1a) selon la revendication 4, dans lequel le fil différent, auquel le deuxième élément d'ancrage (50) est fixé, est l'un de la pluralité de premiers fils (10).

6. Stent auto-expansible (1d) selon l'une quelconque des revendications 4 ou 5, dans lequel le un fil, auquel le troisième élément d'ancrage (70) est fixé, est l'un de la pluralité de premiers fils (10).

7. Stent auto-expansible (1b) selon l'une quelconque des revendications 4 à 6, dans lequel :
une résistance à la traction de chacun de la pluralité de premiers fils (10) est supérieure à une résistance à la traction de chacun de la pluralité de seconds fils (60).

8. Stent auto-expansible (1c) selon l'une quelconque des revendications 1 à 7, dans lequel :
un centre (C) du un fil est déplacé par rapport à un centre (D) du premier élément d'ancrage (20a), comme observé dans une section transversale horizontale, orthogonale à la direction axiale du un fil.

9. Stent auto-expansible (1b) selon la revendication 7, dans lequel :
la pluralité de premiers fils est réalisée à partir d'un alliage de Co-Cr, et
la pluralité de seconds fils est réalisée à partir d'un alliage de Pt-Ni.
